# EUROPEAN PATENT SPECIFICATION

(11) **EP 2 811 915 B1**
(45) Date of publication and mention of the grant of the patent: **26.06.2019**
(21) Application number: 13747042.3
(22) Date of filing: 07.02.2013
(51) Int. Cl.: A61B 17/04

(54) **BONE ANCHOR AND RELATED INSTRUMENTATION**
KNOCHENANKER UND ZUGEHÖRIGE INSTRUMENTE
ANCRE OSSEUSE ET INSTRUMENTS

(30) Priority: 09.02.2012 US 201261596804 P; 09.11.2012 US 201213673626
(43) Date of publication of application: 17.12.2014
(73) Proprietor: Cartiva, Inc., Alpharetta GA 30005 (US)
(72) Inventor: WALES, Lawrence W., Maplewood, Minnesota 55119 (US); PETERS, Jeff, Excelsior, Minnesota 55331 (US); BENTLEY, Ishmael, Eagan, Minnesota 55122 (US)
(74) Representative: Rees, Kerry
(86) International application number: PCT/US2013/025147
(87) International publication number: WO 2013/119812

(56) References cited:
- EP-A1- 1 743 587
- WO-A1-2007/135101
- WO-A2-2010/088561
- US-A- 5 957 953
- US-A1- 2002 133 179
- US-A1- 2010 198 258
- US-A1- 2010 318 125
- US-A1- 2011 004 242

## Description

### TECHNICAL FIELD

The present invention relates to implants and instrumentation for surgical procedures. More specifically, the invention relates to a bone anchor and related suture assemblies and method of using the foregoing.

### BACKGROUND

Various conventional bone anchors are known for use in orthopedic repair procedures. There is a continuing need for improved bone anchors and related instrumentation.

WO2007135101 discloses a tissue anchor for anchoring an expandable valve within the heart. The anchor portion comprises self-deployable prongs and a channel for receiving an attachment mechanism to couple an elongate body portion to the deployed anchor.

WO2010088561 discloses an anchor for attaching tissue to bone that includes an anchor body comprising an axial bore, a plurality of bone- engaging tines extending distally from the anchor body, a spreader comprising an axial bore, and a suture loop extending through the axial bore, wherein the suture loop is secured against being pulled through the axial bore, the spreader is configured to be positioned between the bone-engaging tines, and the spreader is configured to expand the tines outward upon distal or proximal movement of the spreader relative to the anchor body.

### SUMMARY

The present invention discloses an implantable bone anchor assembly and is defined by the features of the independent claim. Preferred embodiments are given in the dependent claims.

While multiple embodiments are disclosed, still other embodiments of the present disclosure will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is an elevation view of a bone anchor assembly according to one embodiment of the present invention.
FIG. 2 is a cross-sectional elevation view of the bone anchor assembly of FIG. 1 according to one embodiment of the present invention.
FIGS. 3-4 are elevation views showing the bone anchor assembly of FIG. 1 during implantation, according to one embodiment of the present invention.
FIGS. 5-10A/B are schematic illustrations of various bone anchor/suture assembly combinations according to various exemplary embodiments of the present disclosure.
FIGS. 11-13 are schematic illustrations of various bone anchor assemblies according to additional exemplary embodiments of the disclosure.
FIG. 14 is a schematic illustration of the skeletal system of a human hand showing exemplary therapeutic applications of various embodiments of the present disclosure.
FIG. 15 is a schematic illustration of the skeletal system of a human foot illustrating exemplary therapeutic applications of various embodiments of the present disclosure.

While the disclosure is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

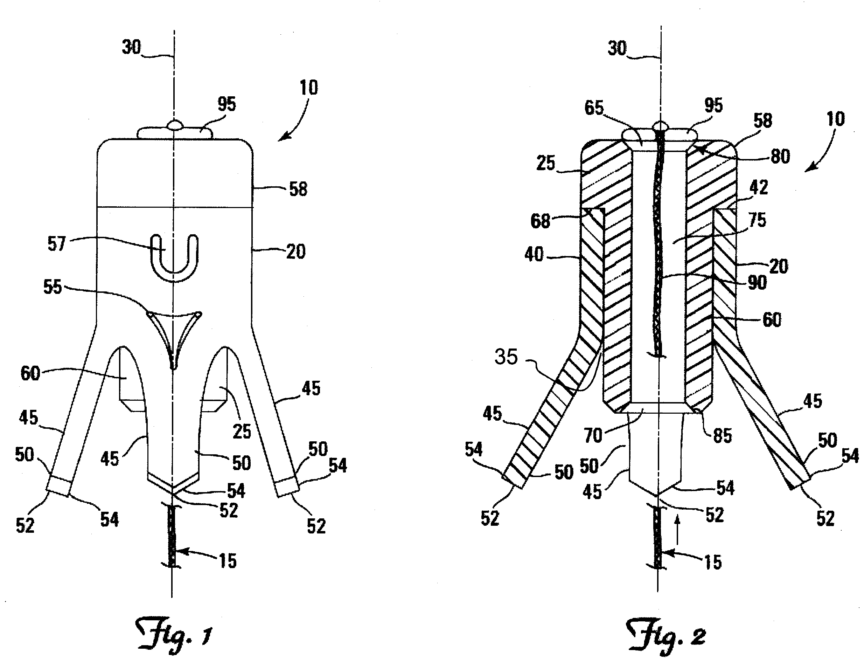

FIGS. 1 and 2 are outer and cross-sectional elevation views of a bone anchor assembly 10 and a suture element 15 according to one embodiment of the present invention. The bone anchor assembly 10 and suture element 15 are, in the various embodiments, particularly suited for orthopedic repair procedures such as, for example, crossover toe, hallux valgus, hammertoe, carpometacarpal arthroplasty, Bankart repair, intervertebral disc repair procedures, or other general orthopedic procedures. As such, the suture element 15 is connected to the bone anchor assembly 10, which when implanted in bone proximate the desired treatment site provides a firm anchoring point to resist tension applied to the suture element 15 both acutely during implantation and chronically after the procedure is complete. In various embodiments, the suture element 15 may include a pre-formed, adjustable suture loop (not shown) that can be interconnected with other suture elements or bone or soft tissue anchors depending on the desired repair technique.

As shown in FIGS. 1 and 2, the bone anchor assembly 10 includes an outer anchor tube 20 and an insert 25. As further shown, the outer anchor tube 20 defines a longitudinal axis 30 of the bone anchor assembly 10, and its tubular shape defines a longitudinal channel 35. In the illustrated embodiment, the outer anchor tube 20 includes a first portion 40 having a first end 42, and a plurality of fingers 45 extending from the first portion 40 generally away from the first end 42. As can be seen in FIGS. 1 and 2, the fingers 45 each have a free end 50 opposite the first portion 40 and terminate in a tip 52 and include edges 54 that are configured to engage bone at the implantation site for securing the bone anchor assembly 10 thereto. Additionally, the first portion 40 includes a plurality of cutouts 55 just proximal to each finger 45 (only one cutout 55 is visible in FIGS. 1 and 2) and a plurality of U-shaped cutouts each forming a tab 57 between the cutouts 55 and the first end 42.

As further shown in FIGS. 1 and 2, each of the fingers 45 is configured to project laterally at an oblique angle relative to the longitudinal axis 30. In addition, the fingers 45 are configured to be deflectable radially inward (i.e., the free ends 50 can be urged toward the longitudinal axis 30) to allow the anchor tube 20 to be disposed within, for example, a tubular cannula during implantation into bone. In the illustrated embodiment, the cutouts 55 operate to provide strain relief in the zone of deflection of the fingers 45. In the illustrated embodiment, four (4) fingers 45 are utilized, although in other embodiments more or fewer than 4 fingers 45 can be present. It will be appreciated that in the various embodiments, the anchor tube 20 includes at least one cutout 55 for each finger 45.

In the illustrated embodiment, the insert 25 includes a head 58 and a shank 60 extending longitudinally from the head 58. As further shown, the insert 25 has a first opening 65 in the head 58, which further includes a radial shoulder 68, a second opening 70 in the shank 60, and a bore 75 extending from the first opening 65 through the second opening 70. In the illustrated embodiment, the first opening 65 is bordered by a chamfered or radiused edge 80, and the second opening 70 is bordered by a chamfered or radiused edge 85.

As shown, the shank 60 is sized so that it can be inserted into the longitudinal channel 35 of the anchor tube 20, with the radial shoulder 68 abutting the first end 42 of the anchor tube 20 first portion 40 when fully inserted. In various embodiments, the fit between the shank 60 and the inner surface of the channel 35 is selected to be sufficiently tight to resist movement of the insert 25 relative to the anchor tube 20. In addition, in the illustrated embodiment, the tabs 57 further operate to engage the insert 25 to further enhance the connection between the insert 25 and the anchor tube 20.

As further shown, the bore 75 is configured to receive the suture element 15, which is configured to be connected to the insert 25. In the illustrated embodiment, the suture element 15 includes a portion 90 disposed within the bore 75 that further includes a pledget 95 having a diameter greater than the diameter of the bore 75 of the insert, such that the suture element 15 cannot readily be pulled proximately through the bore 75 and separated from the bone anchor assembly 10. It is emphasized, however, that the particular technique or structure for connecting the suture element 15 to the insert 25 or the bone anchor assembly 10 is not critical to any of the embodiments of the present invention. For example, in lieu of or in addition to the pledget 95, in various embodiments, a knot, adhesive, or other type of mechanical joining element or technique can be utilized .

The anchor tube 20 and the insert 25 can be made of any number of structurally suitable biocompatible materials. In various embodiments, the anchor tube 20 can be made of a biocompatible alloy or polymeric material. In various embodiments, the anchor tube 20 is made of a superelastic material such as a nickel titanium alloy (e.g., nitinol). Other exemplary materials include titanium, stainless steel, polyetheretherketone, polycarbonate, and combinations thereof. Similarly, the insert 25, in various embodiments, can be made of any number of biocompatible, rigid alloys or polymeric materials, such as titanium, stainless steel, polyetheretherketone, polycarbonate, and combinations thereof. In one embodiment, the anchor tube 20 is made of nitinol, and the insert 25 is made of polyetheretherketone. Various other material combinations can be utilized within the scope of the various embodiments.

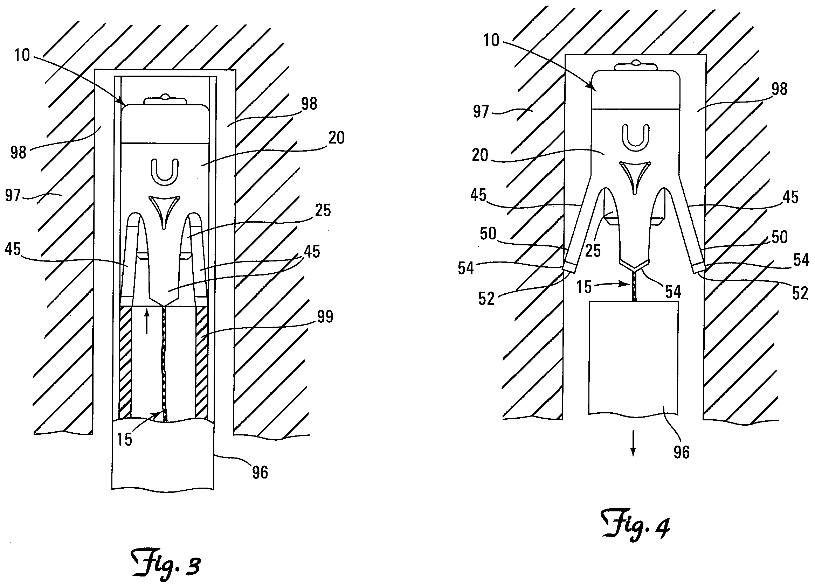

FIGS. 3-4 are elevation views showing implantation of the bone anchor assembly 10 using a cannula 96, according to one embodiment of the present invention. As shown in FIG. 3, in one embodiment, the distal end of the cannula 96, with the bone anchor assembly 10 and suture element 15 disposed therein, is positioned as desired within a bore 98 into the bone 97 at the implantation site. As further shown, when disposed within the cannula 96, the fingers 45 of the anchor tube 20 are deflected radially inward toward the longitudinal axis 30. The bore 98 can be formed by any suitable means, e.g., by use of a bone awl or drill.

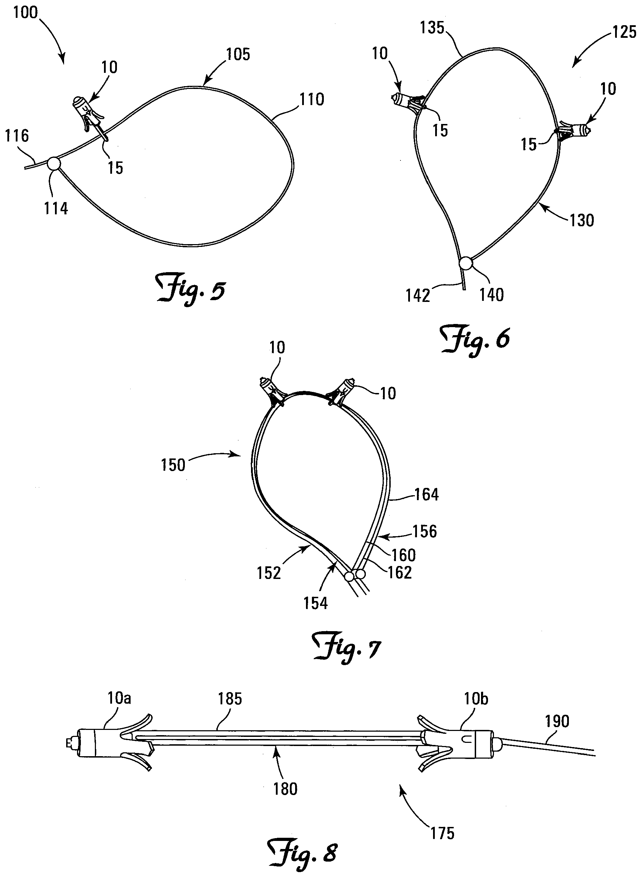

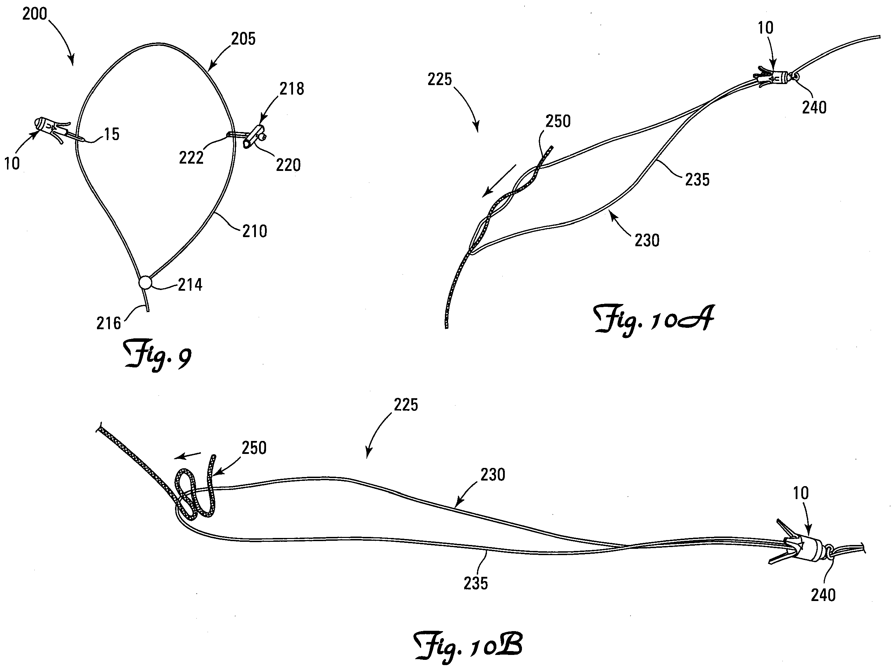

As can be seen in FIG. 4, the cannula 96 can then be withdrawn proximally to release the bone anchor assembly 10 therefrom. For example, a second delivery cannula or push tube 99 can be inserted into the cannula 96 to abut the bone anchor assembly 10 and hold the bone anchor assembly 10 in position while the cannula 96 is withdrawn proximally from the bore 98. As further shown in FIG. 4, once released from the cannula 96, the fingers 45 of the anchor tube 20 self-expand radially outwardly to bear against and engage the bone forming the wall of the bore 98 to secure the bone anchor assembly 10 therein (the engagement of the free end 50, the tip 52 and the edges 54 of the fingers 45 is shown in FIG. 4). Although in FIGS. 3 and 4 a single bone anchor assembly 10 is shown, in various embodiments,as discussed in greater detail below, additional bone anchor assemblies 10 can be disposed serially within the cannula 96 (or other delivery device). In such embodiments, the plurality of bone anchor assemblies 10 can be incorporated into pre-assembled suture assemblies that can take on a variety of configurations for use in various orthopedic procedures. FIGS. 5-10A&B are schematic illustrations of various fixation element constructs according to various exemplary embodiments of the present invention.

FIG. 5 is a schematic illustration of a fixation element 100 according to one embodiment. As shown, the fixation element 100 includes a suture assembly 105, which includes an adjustable suture loop 110, an adjustable knot 114, and a proximal suture length 116 used to tighten the suture loop 110. As further shown, a single bone anchor assembly 10 is slidably coupled to the suture loop 110 by the suture element 15 which is connected to the bone anchor assembly 10 in the manner described above. In the illustrated embodiment, the suture element 15 is in the form of a loop through which the suture material forming the suture loop 110 is passed, thus allowing the bone anchor assembly 10 and suture element 15 to slide along the suture loop 110 as the suture loop is tightened during the particular orthopedic procedure.

FIG. 6 is a schematic illustration of a fixation element 125, which as shown includes a suture assembly 130 having an adjustable suture loop 135, an adjustable knot 140, and a proximal suture length 142. As further shown, a pair of bone anchor assemblies 10 are slidably coupled to the suture loop 135 by respective suture elements 15. Aside from the addition of a second bone anchor assembly 10, the fixation element 125, and its constituent components, operates and is constructed in substantially the same or an identical manner as the fixation element 100. As discussed previously, the fixation element 125 can be pre-loaded into a delivery tool (not shown) with the bone anchor assemblies 10 disposed serially within a delivery cannula, in the manner discussed above with respect to FIGS. 3 and 4.

FIG. 7 is a schematic illustration of an alternative fixation element 150. In the illustrated embodiment, the fixation element 150 includes a pair of bone anchors 10 and corresponding suture elements 15 similar or identical to those discussed previously herein. As further shown, the fixation element 150 includes three suture assemblies 152, 154, 156 each including, respectively, adjustable suture loops 160, 162 and 164, which can each be constructed in substantially the same or a similar manner as the suture loops 110 and 130 discussed above. In the illustrated embodiments, the bone anchor assemblies 10 are slidably coupled to the combined suture loops 160, 162, 164 by their respective suture elements 15. The combination of the three suture assemblies 152, 154, 156 provides a robust, high-strength suture construct.

FIG. 8 is a schematic illustration of another fixation element 175 according to yet another embodiment. As shown in FIG. 8, the fixation element 175 includes a suture assembly 180 and bone anchor assemblies 10a, 10b. In the illustrated embodiment, the suture assembly 180 includes an adjustable suture loop 185 and a proximal suture length 190, which as discussed previously with respect to similar features on other embodiments, can be manipulated to tighten the suture loop 185. The bone anchor assembly 10a is slidably coupled to the suture loop 185, while the anchor assembly 10b is fixedly connected to the suture material making up the suture loop 185 (i.e., cannot slide relative to the suture loop 185). In various embodiments, the bone anchor assemblies 10a, 10b may be disposed, respectively, distally and proximally within a delivery cannula, such that the adjustable bone anchor assembly 10a will be deployed first followed by the fixed bone anchor assembly 10b. In other embodiments, the fixed bone anchor assembly 10b will be disposed distally of the slidable bone anchor assembly 10a in the delivery tool. The specific configuration and orientation of the respective bone anchor assemblies 10a, 10b can be varied depending on the particular orthopedic procedure in which the fixation element 175 is used.

FIG. 9 is a schematic illustration of a fixation element 200 according to one embodiment. As shown, the fixation element 200 includes a suture assembly 205, which includes an adjustable suture loop 210, an adjustable knot 214, and a proximal suture length 216 used to tighten the suture loop 210. As further shown, a single bone anchor assembly 10 is slidably coupled to the suture loop 210 by the suture element 15 which is connected to the bone anchor assembly 10 in the manner described above. As further shown, the fixation element 200 includes a tissue anchor assembly 218 coupled to the suture loop 210. In the illustrated embodiment, the tissue anchor assembly 218 includes a tissue anchor 220 and a suture element 222, which as shown is in the form of a loop through which the suture material forming the suture loop 210 is passed, thus allowing the tissue anchor assembly 218 to slide along the suture loop 210 as it is tightened. In various embodiments, either the bone anchor assembly 10 or the tissue anchor assembly 218 can be fixedly connected to the suture loop 210 in lieu of the slidable coupling arrangement shown. The tissue anchor assembly 218 is configured to be secured to soft tissues (e.g., connective tissue, muscle, or fascia). In various embodiments, the tissue anchor 220 can be formed by a variety of suitable, rigid or semi-rigid polymeric or metallic materials (e.g., polyetheretherketone, PET, titanium, and the like).

FIGS. 10A-10B are schematic illustrations of a fixation element 225 according to yet another embodiment of the present disclosure. As shown, the fixation element 225 includes a suture assembly 230, which includes an adjustable suture loop 235 and an adjustable knot 240. A bone anchor assembly 10 is slidably coupled to the suture loop 235, and a tissue anchor 250 is coupled to the suture loop 235 opposite the adjustable knot 240. The tissue anchor 250 is constructed of suture material, and is formed by passing the suture material forming the suture loop 235 through the suture material of the tissue anchor 250 at multiple locations along the length of the tissue anchor 250. In use, when the suture loop 235 is tightened with the tissue anchor 250 bearing against the tissue to which it is to be secured, the tissue anchor 250 will tend to bunch up and laterally expand, thereby assuming a deployed configuration in which it will bear against the tissue without passing therethroug. FIG. 10A shows the tissue anchor 250 in its initial, undeployed state, while FIG. 10B shows the tissue anchor 250 in its laterally expanded deployed state.

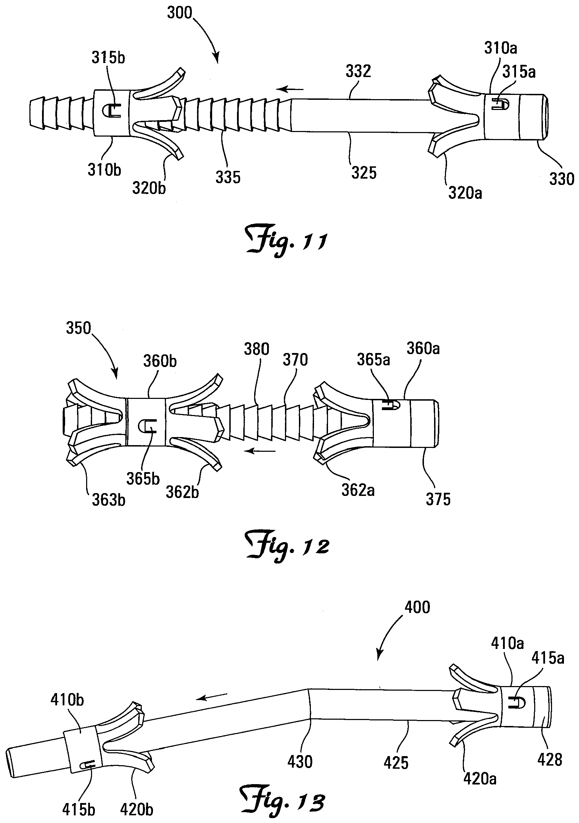

FIG. 11 is an alternative bone anchor assembly 300 according to yet another embodiment of the present disclosure. As shown in FIG. 11, the bone anchor assembly 300 includes a pair of anchor tubes 310a, 310b oriented in opposite directions from one another. Each of the anchor tubes 310a, 310b can be configured in substantially the same or an identical way to the anchor tube 20 of the bone anchor assembly 10. As such, the anchor tube 310a includes at least one tab 315a and plurality of radially deflectable fingers 320a, corresponding to the tab 57 and the fingers 45 of the anchor tube 20. Similarly, the anchor tube 310b includes at least one tab 315b and a plurality of deflectable fingers 320b, also corresponding to the tab 57 and the fingers 45 of the anchor tube 20. As further shown, the bone anchor assembly 300 includes an elongated insert 325 having a head 330, a shank 332 extending longitudinally from the head 330, and a plurality of serrations 335 on a portion of the shank 332. As further shown, shank 332 is disposed within the tubular anchor tubes 310a, 310b. In the illustrated embodiment, the anchor tube 310a is slidable along the shank 332 with the head 330 of the shank 332 delimiting movement of the anchor tube 310a due to the diameter of the head 330 being greater than the inner diameter of the anchor tube 310a.

As further shown, the anchor tube 310b is disposed along the length of the shank 332 including the serrations 335, and is oriented with its fingers 320b facing the fingers 320a of the anchor tube 310a. As can be seen in FIG. 11, each serration includes a surface extending at an oblique angle with respect to the longitudinal axis of the bone anchor assembly 300, and another surface extending generally orthogonal to the longitudinal axis and oriented generally toward the head 330 of the insert 325. Due to the relative orientations of the anchor tubes 310a, 310b, the shank 332 can be pulled through the anchor tube 310b so as to urge the anchor tubes 310a, 310b toward one another (thus applying tension between two bones or bone regions in which the anchor tubes 310a, 310b are embedded. At the same time, reverse movement of the shank 332 is inhibited by engagement of the tab(s) 315b with the serration surface oriented orthogonally to the longitudinal axis. This arrangement allows a desired amount of tension to be maintained between the bones or bone regions to which the anchor tubes 310a, 310b are secured. In the various embodiments, the anchor tubes 310a, 310b and the shank 332 can be made of substantially the same or identical materials as the anchor tube 20 and the insert 25 of the bone anchor assembly 10.

FIG. 12 is an alternative bone anchor assembly 350 according to yet another embodiment of the present disclosure. As shown in FIG. 12, the bone anchor assembly 350 includes a pair of anchor tubes 360a, 360b oriented in opposite directions from one another and each including, respectively, at least one tab 365a, 365b, and a plurality of deflectable fingers 362a, 362b disposed such that the fingers 362a are oriented toward the fingers 362b. As further shown, in the illustrated embodiment, the anchor tube 360b further includes a plurality of deflectable fingers 363b positioned opposite the fingers 362b, and thus oriented in the same general direction as the fingers 362a of the anchor tube 360a. The bone anchor assembly 350 further includes an insert 370 having a head 375 and a shank including a plurality of serrations 380. The insert 370 extends through the anchor tubes 360a, 360b, and the head 375 and the serrations operate in the same manner as the corresponding features of the bone anchor assembly 300 discussed previously.

FIG. 13 is a schematic illustration of a bone anchor assembly 400 according to another embodiment of the present disclosure. As shown, the bone anchor assembly 400 includes a pair of anchor tubes 410a, 410b oriented in opposite directions from one another. Each of the anchor tubes 410a, 410b can be configured in substantially the same or an identical way to the anchor tube 20 of the bone anchor assembly 10. As such, the anchor tube 410a includes at least one tab 415a and plurality of radially deflectable fingers 420a, corresponding to the tab 57 and the fingers 45 of the anchor tube 20. Similarly, the anchor tube 410b includes at least one tab 415b and a plurality of deflectable fingers 420b, also corresponding to the tab 57 and the fingers 45 of the anchor tube 20. As further shown, the bone anchor assembly 400 includes an elongated insert 425 having a head 428, and a shank extending longitudinally from the head 428. The particular shank shown includes a bend 430 at a predetermined location along its length. The bend 430 in the shank provides enhanced flexibility in orienting the bone anchor assembly 400 to provide the desired effect. In various embodiments, the insert 425 can also include serrations (not shown) along its length, similar or identical to the inserts of the bone anchor assemblies previously described. In addition, in some embodiments, the anchor tube 410b can be configured in substantially the same or an identical manner as the anchor tube 360b discussed above (e.g., with two arrangements of deflectable fingers extending in opposite directions from one another).

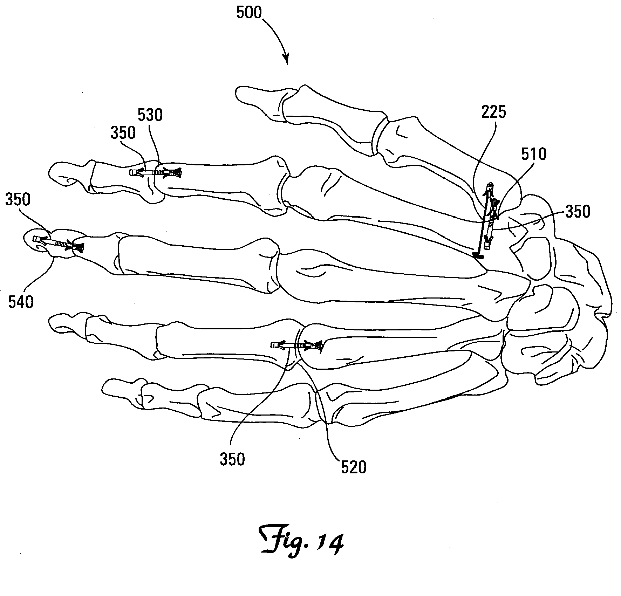

FIG. 14 is a schematic illustration of a model of the skeletal system of a human hand 500 showing exemplary therapeutic applications of various embodiments of the present disclosure. As shown in FIG. 14 at 510, in one exemplary embodiment, a bone anchor assembly 350 can be deployed in combination with a bone anchor/suture assembly - in this case the fixation element 225, in a procedure to repair the carpal metacarpal (CMC) joint. In such an embodiment, a plurality of bone bores can be formed into or through the bones and the bone anchor assembly 360 and the fixation element 225 can be deployed through such bone bores using a suitable delivery tools and techniques, and thereafter tightened to complete the desired orthopedic procedure.

In another example, as further shown in FIG. 14, one of the bone anchor assemblies 350 can also be utilized to accomplish or facilitate fusion of the metacarpophalangeal (MCP) joint 520, the proximal interphalangeal (PIP) joint 530 and/or the distal interphalangeal (DIP) joint 540. In these embodiments, a bone bore can be formed across the respective joints 520, 530, 540 and the bone anchor assembly 350 deployed across the joint through this bore as shown in FIG. 14. In various embodiments, once the bone anchor assembly 350 is inserted into and across the respective joint 520, 530, 540 and the corresponding anchor tubes embedded in the respective bone masses, the insert of the bone anchor assembly 350 can be pulled proximally so as to urge the anchor tubes toward one another thereby accomplishing or aiding in fixation of the joint 520, 530 or 540. It will be appreciated that, in other embodiments, one or more of the additional bone anchor assemblies and/or fixation elements described herein can also be advantageously in the same or similar orthopedic procedures.

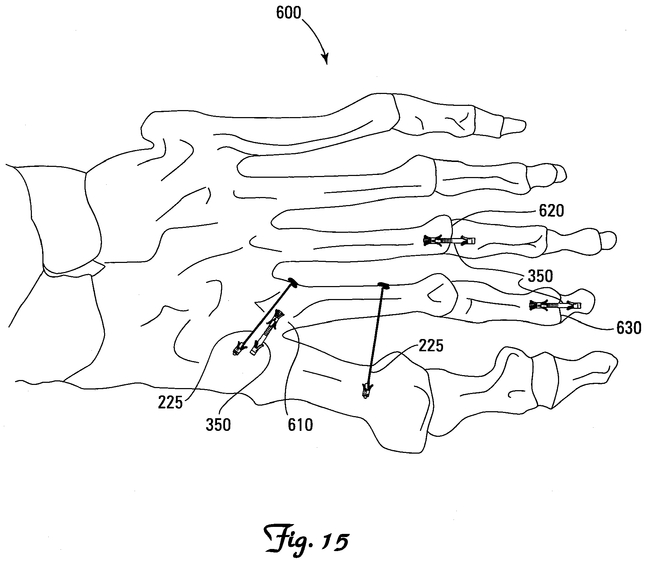

FIG. 15 is a schematic illustration of a model of the skeletal system of a human foot 600 illustrating additional exemplary therapeutic applications of various embodiments of the present disclosure. As shown in FIG. 15 at 610, one or more bone anchor assemblies 350 can be deployed in the bones of the foot 600 in combination with one or more additional anchor/suture assemblies, in this case, the fixation element 225, in hallux valgus and/or hammertoe repair procedures. As further shown in FIG. 15, the bone anchor assembly 350 can also be utilized to accomplish or facilitate fusion of the proximal interphalangeal (PIP) joint 620 and/or the distal interphalangeal (DIP) joint 630 of the foot 600. In these embodiments, a bone bore can be formed across the respective joints 620, 630 and the bone anchor assembly 350 deployed across the joint through this bore. In various embodiments, once the bone anchor assembly 350 is inserted into and across the respective joint 620, 630 and the corresponding anchor tubes embedded in the respective bone masses, the insert of the bone anchor assembly 350 can be pulled proximally so as to urge the anchor tubes toward one another thereby accomplishing or aiding in fixation of the joint 620 or 630, as the case may be. It will be appreciated that, in other embodiments, one or more of the additional bone anchor assemblies and/or fixation elements described herein can also be advantageously in the same or similar orthopedic procedures.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. An implantable bone anchor assembly (10) comprising:
an outer anchor tube (20) defining a longitudinal axis (30) of the bone anchor assembly and a longitudinal channel (35), the outer anchor tube comprising a plurality of fingers (45) circumferentially spaced from one another about the longitudinal axis, each of the fingers having a free end (50);
wherein the fingers are configured to project laterally outwardly at an angle relative to the longitudinal axis; and
wherein the fingers are configured to self-expand radially outwardly and are configured to be deflectable radially inwardly for deployment; and
an insert (25) comprising a bore (75), the insert comprising a head (58) and a shank (60), wherein the shank extends through the longitudinal channel of the outer anchor tube, wherein the bore of the insert extends from an opening in the head of the insert to a second opening (70) in the shank located generally opposite the head;
wherein the outer anchor tube is secured to the insert by insertion of the shank into the longitudinal channel of the outer anchor tube; **characterised in that**
a suture element (15) extends within the bore of the insert from the opening in the head of the insert to and proximally through the second opening, wherein the suture element is configured to engage the insert and resist withdrawal of the suture element from the bore of the insert after implantation.

2. The bone anchor assembly of Claim 1, wherein the head of the insert comprises a shoulder (68) that is configured to abut the outer anchor tube when the shank is fully inserted into the longitudinal channel of the outer anchor tube, wherein a fit between the shank and inner surface of the longitudinal channel is tight to resist movement of the insert relative to the anchor tube.

3. The bone anchor assembly of Claim 1 or 2, wherein the outer anchor tube comprises a plurality of tabs (57) configured to engage the insert to further enhance a connection between the insert and the outer anchor tube when the shank of the insert is inserted into the longitudinal channel of the outer anchor tube.

4. A bone anchor assembly according to any one of the preceding claims, wherein the outer anchor tube comprises a shape memory alloy.

5. A bone anchor assembly according to any one of the preceding claims, wherein the insert comprises polyetheretherketone.

6. A bone anchor assembly according to any preceding claim, wherein the fingers are configured to project laterally at an oblique angle relative to the longitudinal axis.

7. A bone anchor assembly according to any preceding claim, wherein each of the fingers terminates in a tip (52) and includes edges (54) configured to engage bone at an implantation site for securing the bone anchor assembly thereto.

8. A bone anchor assembly according to any one of Claims 1 to 3, wherein the suture element forms a loop through which a separate suture loop is passed.

9. The bone anchor assembly of Claim 8, further comprising the separate suture loop, wherein the separate suture loop comprises an adjustable suture loop, an adjustable knot and a proximal suture length to tighten the adjustable suture loop.

10. The bone anchor assembly of Claim 9, wherein the separate suture loop is configured to secure to a second bone anchor.

11. A bone anchor assembly according to any one of Claims 1 to 3, wherein the suture element is configured to engage the insert and resist withdrawal of the suture element from the bore of the insert using a pledget.

12. A bone anchor assembly according to any one of Claims 1 to 3, wherein the suture element is configured to engage the insert and resist withdrawal of the suture element from the bore of the insert using at least one of a knot, an adhesive and another type of mechanical joining element.

13. A bone anchor assembly according to Claim 1, wherein:
the head of the insert comprises a shoulder that is configured to abut the outer anchor tube when the shank is fully inserted into the longitudinal channel of the outer anchor tube, wherein a fit between the shank and inner surface of the longitudinal channel is tight to resist movement of the insert relative to the anchor tube;
the outer anchor tube comprises a shape memory alloy;
the insert comprises polyetheretherketone;
the fingers are configured to project laterally at an oblique angle relative to the longitudinal axis;
the suture element forms a loop through which a separate suture loop is passed; and
the suture element is configured to engage the insert and resist withdrawal of the suture element from the bore of the insert using at least one of a pledget, a knot, an adhesive and another type of mechanical joining element.

## Patentansprüche

1. Implantierbare Knochenankeranordnung (10), die Folgendes umfasst:
eine äußere Ankerröhre (20), die eine Längsachse (30) der Knochenankeranordnung und einen längslaufenden Durchgang (35) definiert, wobei die äußere Ankerröhre eine Vielzahl von Fingern (45) umfasst, die in Umfangsrichtung um die Längsachse herum voneinander beabstandet angeordnet sind, wobei jeder der Finger ein freies Ende (50) aufweist;
wobei die Finger dazu konfiguriert sind, unter einem Winkel relativ zu der Längsachse lateral nach außen vorzustehen; und
wobei die Finger dazu konfiguriert sind, sich von selbst radial nach außen zu spreizen und dazu konfiguriert sind, zum Einbringen radial nach innen auslenkbar zu sein; und
einen eine Bohrung (75) umfassenden Einsatz (25), wobei der Einsatz einen Kopf (58) und einen Schaft (60) aufweist, wobei sich der Schaft durch den längslaufenden Durchgang der äußeren Ankerröhre erstreckt, wobei sich die Bohrung des Einsatzes von einer Öffnung in dem Kopf des Einsatzes zu einer allgemein dem Kopf gegenüberliegenden zweiten Öffnung (70) in dem Schaft erstreckt;
wobei die äußere Ankerröhre durch Einsetzen des Schafts in den längslaufenden Durchgang der äußeren Ankerröhre an dem Einsatz fixiert wird; **dadurch gekennzeichnet, dass**
sich ein Fadenelement (15) in der Bohrung des Einsatzes von der Öffnung in dem Kopf des Einsatzes zu der und proximal durch die zweite Öffnung erstreckt, wobei das Fadenelement dazu konfiguriert ist, mit dem Einsatz in Eingriff zu gelangen und dem Herausziehen des Fadenelements aus der Bohrung des Einsatzes nach der Implantation zu widerstehen.

2. Knochenankeranordnung nach Anspruch 1, wobei der Kopf des Einsatzes eine Flanke (68) umfasst, die dazu konfiguriert ist, an der äußeren Ankerröhre anzuliegen, wenn der Schaft ganz in den längslaufenden Kanal der äußeren Ankerröhre eingesetzt ist, wobei eine Passung zwischen dem Schaft und der inneren Oberfläche des längslaufenden Durchgangs eng ist, um der Bewegung des Einsatzes relativ zu der Ankerröhre zu widerstehen.

3. Knochenankeranordnung nach Anspruch 1 oder 2, wobei die äußere Ankerröhre eine Vielzahl von Nasen (57) umfasst, die dazu konfiguriert sind, mit dem Einsatz in Eingriff zu gelangen, um eine Verbindung zwischen dem Einsatz und der äußeren Ankerröhre weiter zu verstärken, wenn der Schaft des Einsatzes in den längslaufenden Durchgang der äußeren Ankerröhre eingesetzt wird.

4. Knochenankeranordnung nach einem der vorangehenden Ansprüche, wobei die äußere Ankerröhre eine Formgedächtnislegierung umfasst.

5. Knochenankeranordnung nach einem der vorangehenden Ansprüche, wobei der Einsatz Polyetheretherketon umfasst.

6. Knochenankeranordnung nach einem der vorangehenden Ansprüche, wobei die Finger dazu konfiguriert sind, unter einem schiefen Winkel relativ zu der Längsachse lateral vorzustehen.

7. Knochenankeranordnung nach einem der vorangehenden Ansprüche, wobei jeder der Finger in einer Spitze (52) endet und Kanten (54) umfasst, die dazu konfiguriert sind, an einer Implantationsstelle mit Knochen in Eingriff zu gelangen, um die Knochenankeranordnung daran zu fixieren.

8. Knochenankeranordnung nach einem der Ansprüche 1 bis 3, wobei das Fadenelement eine Schlinge bildet, durch die eine separate Fadenschlinge geführt ist.

9. Knochenankeranordnung nach Anspruch 8, weiter umfassend die separate Fadenschlinge, wobei die separate Fadenschlinge eine verstellbare Fadenschlinge, einen verstellbaren Knoten und ein proximales Fadenstück zum Festziehen der verstellbaren Fadenschlinge umfasst.

10. Knochenankeranordnung nach Anspruch 9, wobei die separate Fadenschlinge zum Fixieren an einem zweiten Knochenanker konfiguriert ist.

11. Knochenankeranordnung nach einem der Ansprüche 1 bis 3, wobei das Fadenelement dazu konfiguriert ist, mit dem Einsatz in Eingriff zu gelangen und dem Herausziehen des Fadenelements aus der Bohrung des Einsatzes unter Verwendung eines Tupfers zu wiederstehen.

12. Knochenankeranordnung nach einem der Ansprüche 1 bis 3, wobei das Fadenelement dazu konfiguriert ist, mit dem Einsatz in Eingriff zu gelangen und dem Herausziehen des Fadenelements aus der Bohrung des Einsatzes unter Verwendung mindestens eines von einem Knoten, einem Klebstoff und einer anderen Art von mechanischem Verbindungelement zu wiederstehen.

13. Knochenankeranordnung nach Anspruch 1, wobei:
der Kopf des Einsatzes eine Flanke umfasst, die dazu konfiguriert ist, an der äußeren Ankerröhre anzuliegen, wenn der Schaft ganz in den längslaufenden Kanal der äußeren Ankerröhre eingesetzt ist, wobei eine Passung zwischen dem Schaft und der inneren Oberfläche des längslaufenden Durchgangs eng ist, um der Bewegung des Einsatzes relativ zu der Ankerröhre zu widerstehen;
die äußere Ankerröhre eine Formgedächtnislegierung umfasst;
der Einsatz Polyetheretherketon umfasst;
die Finger dazu konfiguriert sind, unter einem schiefen Winkel relativ zu der Längsachse lateral vorzustehen;
das Fadenelement eine Schlinge bildet, durch die eine separate Fadenschlinge geführt ist; und
das Fadenelement dazu konfiguriert ist, mit dem Einsatz in Eingriff zu gelangen und dem Herausziehen des Fadenelements aus der Bohrung des Einsatzes unter Verwendung mindestens eines von einem Tupfer, einem Knoten, einem Klebstoff und einer anderen Art von mechanischem Verbindungselement zu widerstehen.

## Revendications

1. Ensemble d'ancrage osseux implantable (10) comprenant :
un tube d'ancrage externe (20) définissant un axe longitudinal (30) de l'ensemble d'ancrage osseux et un canal longitudinal (35), le tube d'ancrage externe comprenant une pluralité de doigts (45) espacés les uns des autres de manière circonférentielle autour de l'axe longitudinal, chacun des doigts ayant une extrémité libre (50) ;
dans lequel les doigts sont configurés pour une projection latérale vers l'extérieur selon un angle par rapport à l'axe longitudinal ; et
dans lequel les doigts sont configurés pour une auto-expansion radiale vers l'extérieur et sont configurés pour pouvoir être déviés radialement vers l'intérieur pour un déploiement ; et
un insert (25) comprenant un alésage (75), l'insert comprenant une tête (58) et une tige (60), la tige s'étendant à travers le canal longitudinal du tube d'ancrage externe, l'alésage de l'insert s'étendant d'une ouverture dans la tête de l'insert à une deuxième ouverture (70) dans la tige située généralement en regard de la tête ;
dans lequel le tube d'ancrage externe est fixé à l'insert par insertion de la tige dans le canal longitudinal du tube d'ancrage externe ;
**caractérisé en ce qu'**un élément de suture (15) s'étend à l'intérieur de l'alésage de l'insert de l'ouverture dans la tête de l'insert à la seconde ouverture et de manière proximale à celle-ci, l'élément de suture étant configuré pour venir en prise avec l'insert et résister à un retrait de l'élément de suture à partir de l'alésage de l'insert après implantation.

2. Ensemble d'ancrage osseux selon la revendication 1, dans lequel la tête de l'insert comprend un épaulement (68) qui est configuré pour venir en butée contre le tube d'ancrage externe lorsque la tige est entièrement insérée dans le canal longitudinal du tube d'ancrage externe, dans lequel un ajustement entre la tige et la surface interne du canal longitudinal est serré afin de résister à un mouvement de l'insert par rapport au tube d'ancrage.

3. Ensemble d'ancrage osseux selon la revendication 1 ou 2, dans lequel le tube d'ancrage externe comprend une pluralité de languettes (57) configurées pour venir en prise avec l'insert afin d'améliorer encore une connexion entre l'insert et le tube d'ancrage externe lorsque la tige de l'insert est insérée dans le canal longitudinal du tube d'ancrage externe.

4. Ensemble d'ancrage osseux selon l'une quelconque des revendications précédentes, dans lequel le tube d'ancrage externe comprend un alliage à mémoire de forme.

5. Ensemble d'ancrage osseux selon l'une quelconque des revendications précédentes, dans lequel l'insert comprend de la polyétheréthercétone.

6. Ensemble d'ancrage osseux selon l'une quelconque des revendications précédentes, dans lequel les doigts sont configurés pour une projection latérale selon un angle oblique par rapport à l'axe longitudinal.

7. Ensemble d'ancrage osseux selon l'une quelconque des revendications précédentes, dans lequel chacun des doigts se termine en une pointe (52) et inclut des bords (54) configurés pour venir en prise avec l'os au niveau d'un site d'implantation afin de fixer l'ensemble d'ancrage osseux à celui-ci.

8. Ensemble d'ancrage osseux selon l'une quelconque des revendications 1 à 3, dans lequel l'élément de suture forme une boucle à travers laquelle une boucle de suture distincte est amenée à passer.

9. Ensemble d'ancrage osseux selon la revendication 8, comprenant en outre la boucle de suture distincte, dans lequel la boucle de suture distincte comprend une boucle de suture réglable, un noeud réglable et une longueur de suture proximale afin de serrer la boucle de suture réglable.

10. Ensemble d'ancrage osseux selon la revendication 9, dans lequel la boucle de suture distincte est configurée pour une fixation à un deuxième ancrage osseux.

11. Ensemble d'ancrage osseux selon l'une quelconque des revendications 1 à 3, dans lequel l'élément de suture est configuré pour venir en prise avec l'insert et résister à un retrait de l'élément de suture à partir de l'alésage de l'insert à l'aide d'un tampon.

12. Ensemble d'ancrage osseux selon l'une quelconque des revendications 1 à 3, dans lequel l'élément de suture est configuré pour venir en prise avec l'insert et résister à un retrait de l'élément de suture à partir de l'alésage de l'insert à l'aide d'au moins l'un parmi un noeud, un adhésif et un autre type d'élément d'assemblage mécanique.

13. Ensemble d'ancrage osseux selon la revendication 1, dans lequel :
la tête de l'insert comprend un épaulement qui est configuré pour venir en butée contre le tube d'ancrage externe lorsque la tige est entièrement insérée dans le canal longitudinal du tube d'ancrage externe, dans lequel un ajustement entre la tige et la surface interne du canal longitudinal est serré afin de résister à un mouvement de l'insert par rapport au tube d'ancrage ;
le tube d'ancrage externe comprend un alliage à mémoire de forme ;
l'insert comprend de la polyétheréthercétone ;
les doigts sont configurés pour une projection latérale selon un angle oblique par rapport à l'axe longitudinal ;
l'élément de suture forme une boucle à travers laquelle une boucle de suture distincte est amenée à passer ; et
l'élément de suture est configuré pour venir en prise avec l'insert et résister à un retrait de l'élément de suture à partir de l'alésage de l'insert à l'aide d'au moins l'un parmi un tampon, un noeud, un adhésif et un autre type d'élément d'assemblage mécanique.
